# EUROPEAN PATENT APPLICATION

(11) **EP 2 394 736 A1**
(43) Date of publication of application: **14.12.2011**
(21) Application number: 11169901.3
(22) Date of filing: 14.06.2011
(51) Int. Cl.: B01J 20/28, B01D 15/00, A61M 1/36

(54) **Adsorbent device for the treatment of biological fluids**

(30) Priority: 14.06.2010 IT BO20100376
(71) Applicant: Medica S.p.A., Medolla (MO) (IT)
(72) Inventor: Palumbo, Giuseppe, 41121 Modena (IT); Bocchi, Letizia, 41036 Medolla (IT); Fecondini, Luciano, 40129 Bologna (IT)
(74) Representative: Jorio, Paolo

(57) **Abstract**

An adsorbent filter (1; 11) for treatment of biological fluids constituted by a supporting structure (2), made in which are an adsorption chamber (4) and at least one pair of openings respectively for inlet and outlet of the fluid to be treated into and from the adsorption chamber (4), and an adsorbent material (7; 7a, 7b, 7c) housed in the adsorbent chamber. The adsorbent material (7; 7a, 7b, 7c) comprises fragments of hollow adsorbent fibre not constrained at the ends. The fragments of adsorbent fibre are made of a material comprised in the group constituted by polysulphone and its derivatives, cellulose and its derivatives, and polyacrylonitrile, and have a length comprised between 5 mm and 20 mm.

## Description

The present invention relates to an adsorbent device for the treatment of biological fluids.

The potentialities of adsorbent devices have been known for some time now.

Such devices, in fact, are used in various therapeutic applications, such as for example for treatment of auto-immune inflammatory pathological conditions, in which the inflammatory process is generated by the increase in number and activity of monocytes, neutrophilic granulocytes, and pro-inflammatory cytokines.

An effective extracorporeal removal of neutrophilic granulocytes and monocytes enables a transient reduction thereof with a positive effect on the inflammatory process.

The aim of the present invention is to provide an adsorbent device that will find an effective application in therapies for purification of biological fluids, such as, for example, blood.

The subject of the present invention is use of fragments of adsorbent hollow fibre not constrained at the ends for the treatment of biological fluids, the basic characteristics of which appear in Claim 1 and the preferred and/or auxiliary characteristics of which appear in Claims 2 and 3.

A further subject of the present invention is an adsorbent filter for treatment of biological fluids, the basic characteristics of which appear in Claim 4 and the preferred and/or auxiliary characteristics of which appear in Claims 5-7.

For a better understanding of the invention embodiments thereof are provided hereinafter purely by way of illustrative and non-limiting example with the aid of the figures of the annexed plate of drawings, wherein:
Figure 1 is a side view with parts removed for simplicity of a first embodiment of the device forming the subject of the present invention;
Figure 2 is a side view with parts removed for simplicity of a second embodiment of the device forming the subject of the present invention; and
Figure 3 is a graph representing tests of adsorption of the device forming the subject of the present invention.

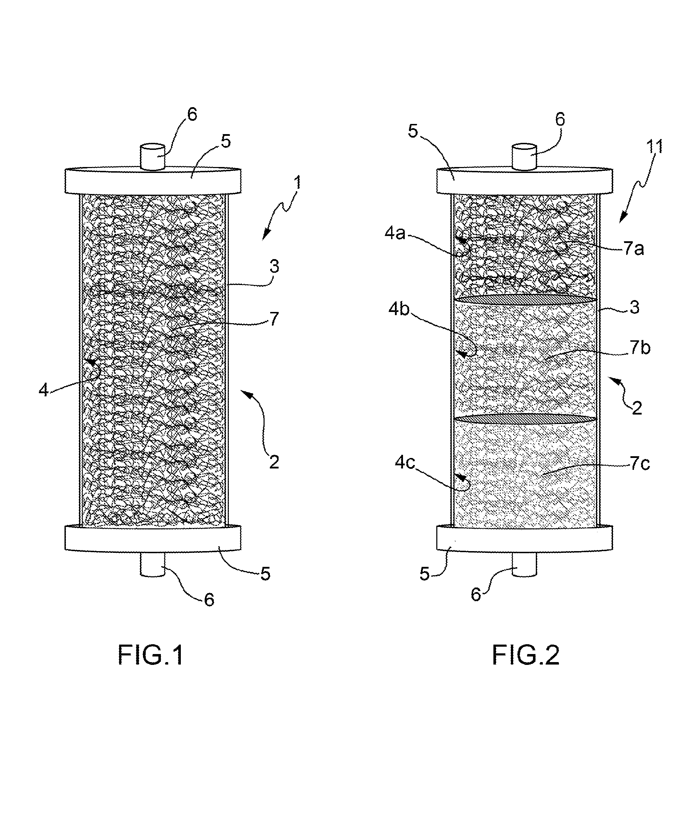

Designated as a whole by 1 in Figure 1 is the adsorbent device forming the subject of the present invention.

The device 1 comprises a cartridge 2, comprising a side wall 3 of a cylindrical shape, illustrated in cross section for reasons of clarity of description, and designed to define an adsorption chamber 4. The cartridge 2 further comprises a pair of circular closing elements 5 arranged so as to close the axial ends of the side wall 3. In each of the circular closing elements 5 an opening is made for enabling inlet and outlet of the fluid to be treated. Finally, each of the circular closing elements 5 comprises a connector 6 set in a position corresponding to the respective opening for connection of treatment tubes. Generally, the cartridge 2 is made of plastic material, such as, for example, polycarbonate.

The openings for inlet and outlet of the fluid to be treated can also be made in the lateral surface of the cartridge.

Housed inside the adsorption chamber is an adsorbent material 7 constituted by fragments of hollow fibre not constrained at the ends. The fragments preferably have a length smaller than or equal to 30 mm, and still more preferably between 2 and 20 mm.

The hollow fibres can be made of polysulphone and its derivatives, cellulose and its derivatives, polyacrylonitrile and polymers of natural or synthetic origin designed to be used for bestowing selectivity for given ions, molecules, or target cells.

The hollow fibres generally have an inner diameter comprised between 150 µm and 3000 µm, preferably between 180 µm and 300 µm.

Provided hereinafter are three examples of application of the device forming the subject of the present invention.

### - ADSORPTION ON BLOOD -

An adsorbent device was produced as described above. In particular, the device comprises a polycarbonate cartridge, having a diameter of 38 mm and a length of 180 mm. The adsorption chamber was filled with 17 grams of fragments of hollow polysulphone fibres. The fragments had a length ranging from 5 to 20 mm. The hollow fibre presented a cut-off comprised between 15 and 50 kDa.

The adsorbent capacity of the device thus prepared was tested on whole blood taken from a healthy donor.

The blood was passed, with a current flowrate (without recirculation) of 30 ml/min, through the device and the adsorption of various types of cells was evaluated via periodic sampling of eluate. In particular, samples of eluate were taken at 10, 20, and 30 minutes.

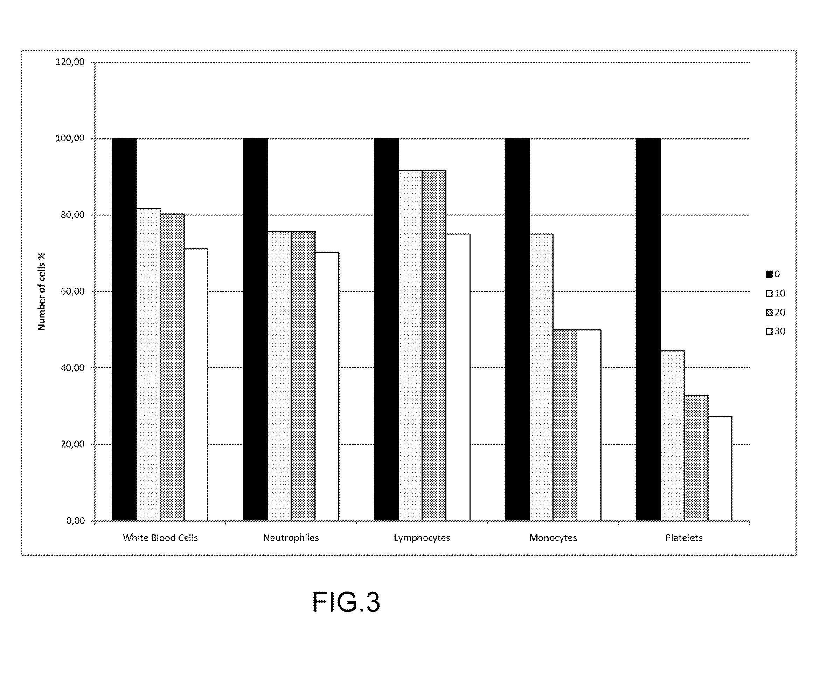

The values appearing in the graph of Figure 3 present a normalization at 100 of the value of the various types of cells detected in a sample taken upstream of the device.

As may be noted from the data appearing in the graph of Figure 3, the device produced in time: an adsorption of approximately 30-50% of the number of monocytes; an adsorption of 25-30% of the number of neutrophilic granulocytes; an adsorption of 50-70% of the number of platelets; and an adsorption of 20-30% of the total number of leukocytes. In parallel, there were noted minimal fluctuations of the lymphocytes (10-25%) and no variation of erythrocytes, haemoglobin, and haematocrit.

As may be noted from the data given above, the adsorbent device according to the present invention enables an efficient action of adsorption of the cells in the biological fluids to be treated, such as, for example, blood.

In addition, the effect of adsorption is absolutely independent of whether the hollow fibres are closed or open.

Illustrated and designated as a whole by 11 in Figure 2 is a second embodiment of the adsorbent device forming the subject of the present invention. The parts of the device 11 that are the same as those of the device 1 described above will be designated by the same numbers and will not be described again.

The device 11 differs from the device 1 owing to the presence of two partitioning disks 12, which are made of a material suitable for enabling passage of the fluid to be treated and are set within the adsorption chamber 4 to divide it into three subchambers 4a, 4b, 4c. Housed in each of the subchambers is a respective type of adsorbent material 7a, 7b, 7c having the same characteristics as those given above for the adsorbent material 7 of the device 1. In other words, the adsorbent material used in the device 11 is made with fragments of hollow fibre of a length equal to or smaller than 30 mm, preferably comprised between 5 and 20 mm. The hollow fibres are made of the same materials as those given above for the adsorbent material 7.

The adsorbent device 11 affords the advantage of enabling simultaneous use of different adsorbent materials, each of which is specific for a given ion, molecule, or target cell. In this way, it will be possible to provide a simultaneous removal of different compounds by treatment with a single device.

Finally, it is possible to provide an adsorbent device according to the present invention in which in one and same adsorption chamber the adsorbent material is made up of a number of types of adsorbent material. Also in this way, it will be possible to provide a simultaneous removal of different compounds by treatment with a single device without on the other hand dividing the adsorption chamber.

## Claims

1. Use of fragments of hollow adsorbent fibre not constrained at the ends for the treatment of biological fluids; said adsorbent fibre being made of a material comprised in the group constituted by polysulphone and its derivatives, cellulose and its derivatives, and polyacrylonitrile; said fragments of hollow fibre having a length comprised between 5 and 20 mm.

2. Use according to Claim 1, **characterized in that** said fragments have an inner diameter comprised between 150 µm and 3000 µm.

3. Use according to Claim 2, **characterized in that** said fragments have an inner diameter comprised between 180 µm and 300 µm.

4. An adsorbent filter (1; 11) for treatment of biological fluids, comprising a supporting structure (2) made in which are an adsorption chamber (4) and at least one pair of openings, respectively for inlet and outlet of the fluid to be treated into and from the adsorption chamber (4), and an adsorbent material (7; 7a, 7b, 7c) housed in said adsorbent chamber; said adsorbent filter being **characterized in that**:
said adsorbent material (7; 7a, 7b, 7c) comprises fragments of hollow adsorbent fibre not constrained at the ends; said fragments of adsorbent fibre are made of a material comprised in the group constituted by polysulphone and its derivatives, cellulose and its derivatives, and polyacrylonitrile, and have a length comprised between 5 and 20 mm.

5. The adsorbent filter (1; 11) according to Claim 4, **characterized in that** said fragments have an inner diameter comprised between 150 µm and 3000 µm.

6. The adsorbent filter (1; 11) according to Claim 5, **characterized in that** said fragments have an inner diameter comprised between. 180 µm and 300 µm.

7. The adsorbent filter (11) according to any one of the preceding claims, **characterized in that** it comprises one or more partitioning elements (12) designed to be housed within said adsorption chamber (4).

8. A method for extracorporeal treatment of biological fluids, **characterized in that** it comprises a step of adsorption through an adsorbent filter; said method being **characterized in that** said adsorbent filter is of the type claimed in any one of Claims 4 to 7.
